# EUROPEAN PATENT APPLICATION

(11) **EP 0 661 079 A1**
(43) Date of publication of application: **05.07.1995**
(21) Application number: 93916201.2
(22) Date of filing: 21.07.1993
(51) Int. Cl.: A61N 2/02

(54) **MAGNETOTHERAPY APPARATUS**

(71) Applicant: NIHONKENKOZOSHINKENKYUKAI CO. LTD., Fukuoka-shi Fukuoka 813 (JP)
(72) Inventor: MASUDA, Isamu,c/o Nihonkenkozoshinkenkyukai Co.Ltd, Fukuoka-shi, Fukuoka 813 (JP)
(74) Representative: Dipl.-Phys.Dr. Manitz Dipl.-Ing. Finsterwald Dipl.-Ing. Grämkow Dipl.-Chem.Dr. Heyn Dipl.-Phys. Rotermund Morgan B.Sc.(Phys.)
(86) International application number: JP9301018
(87) International publication number: WO9503088

(57) **Abstract**

A magnetotherapy apparatus used to subject a patient to magnetotherapy by applying a magnetic field to a wide rage of the surface of his body. This apparatus consists of a combination of a mounting base, and a plurality of magnetic field generating members (10) each of which is composed of a plurality of cases, in each of which a solenoid is housed, which are arranged with like magnetic pole surfaces thereof facing in the same direction, and which are connected together in a row so that they can be bent with respect to one another, these magnetic field generating members (10) being arranged in parallel with one another on the mounting base with the like magnetic pole surfaces thereof positioned in the same direction. The magnetic field generating members (10) generate vibration and heat in addition to magnetic fields, which work on a wide range of the surface of a human body, whereby excellent therapeutic effects are obtained.

## Description

### Technical Field

This invention relates to a magnetic therapeutic apparatus which performs magnetic therapy by exerting magnetic fields over a wide range of the human body.

### Background Art

The applicant of this invention formerly proposed a magnetic therapeutic apparatus formed by flexibly connecting in one row, a plurality of cases each housing solenoids therein, with their magnetic pole faces orienting in the same direction. After this magnetic therapeutic apparatus is installed by winding it around a diseased part of a human body, when A.C. current is flowed through the solenoids of each case, an alternating magnetic field generated from each solenoid acts on the human body, and thereby various symptoms such as stiffness are alleviated. Furthermore, this magnetic therapeutic apparatus generates vibration and heat due to current flowing to the solenoids, thereby providing the human body with a massaging effect as well as a warming effect.

However, this type of magnetic therapeutic apparatus is so structured that cases containing solenoids are connected in one row, thus there is a problem that even when the apparatus is installed so that it's wound around the human body, an effective range of the magnetic fields is limited to a narrow scope.

Besides this type of magnetic therapeutic apparatus, a cushion mat for the magnetic therapy produced by magnetizing a cushion material containing magnetic powder, has also been proposed. When lying on this cushion mat, D.C. magnetic fields generate all over the cushion mat, and a range of the magnetic fields acting on the human body becomes wide, while the magnetic fields from the cushion mat permanently magnetized are insufficient in intensity and therefore the cushion mat lacks a sufficient effect of the magnetic therapy, and magnetic intensity decreases with the lapse of time, thus the mat cannot be used for a long time. Moreover, the cushion does not generate vibration and heat, and the massaging effect or the warming effect can not be obtained.

### Disclosure of Invention

A magnetic therapeutic apparatus of this invention comprises a mounting base and a plurality of magnetic field generators which are disposed in parallel on the mounting base, with their magnetic pole faces aligning, and said each magnetic field generator is so structured that a plurality of cases each containing solenoids therein are flexibly connected in one row with their magnetic pole orienting in the same direction.

According to this invention, the magnetic pole faces are arranged in two dimensions, thus a range of the magnetic fields acting on the human body becomes wide and a remarkable effect of the magnetic therapy can be expected.

Furthermore, the magnetic field is generated by flowing current in the solenoids, thus the magnetic field intensity can be strengthened as compared with a D.C. magnetic field from a permanently magnetized cushion sheet, and the effect of the magnetic therapy can be enhanced, in addition the problem of demagnetization with the elapse of time fails to occur. Moreover, vibration and heat are generated due to flowing current in the solenoid, thus the massaging and warming effects as well as the effect of the magnetic therapy can be obtained over a wide range.

Furthermore, the cases of each magnetic field generator are flexibly connected with each other, thus they can be disposed along the shape of the mounting base, and only a necessary number of the cases can be mounted depending upon an area of the mounting base.

### Brief Description of the Drawings

Fig. 1 is a perspective view showing a magnetic therapeutic apparatus according to first embodiment of this invention.

Fig. 2 is a side view showing a state of use of the embodiment of Fig. 1.

Fig. 3 is a plan view showing a magnetic field generator provided in a magnetic therapeutic apparatus.

Fig. 4 is a longitudinal section view showing a magnetic field generator.

Fig. 5 is a front view showing a structure of a solenoid.

Fig. 6 is a rear view showing a magnetic therapeutic apparatus.

Fig. 7 is a front view showing a magnetic therapeutic apparatus.

Fig. 8 is a bottom view showing a magnetic therapeutic apparatus.

Fig. 9 is a longitudinal section view of a bed showing a state of installation of magnetic field generators.

Fig. 10 is a view showing a state of installation of magnetic field generators in a head support.

Fig. 11 is a longitudinal section view showing a cover.

Fig. 12 is a longitudinal section view showing a foot support.

Fig. 13 is a plan view showing a magnetic therapeutic apparatus according to the second embodiment of this invention.

Fig. 14 is a side view of the second embodiment.

Fig. 15 is a longitudinal section view of the second embodiment.

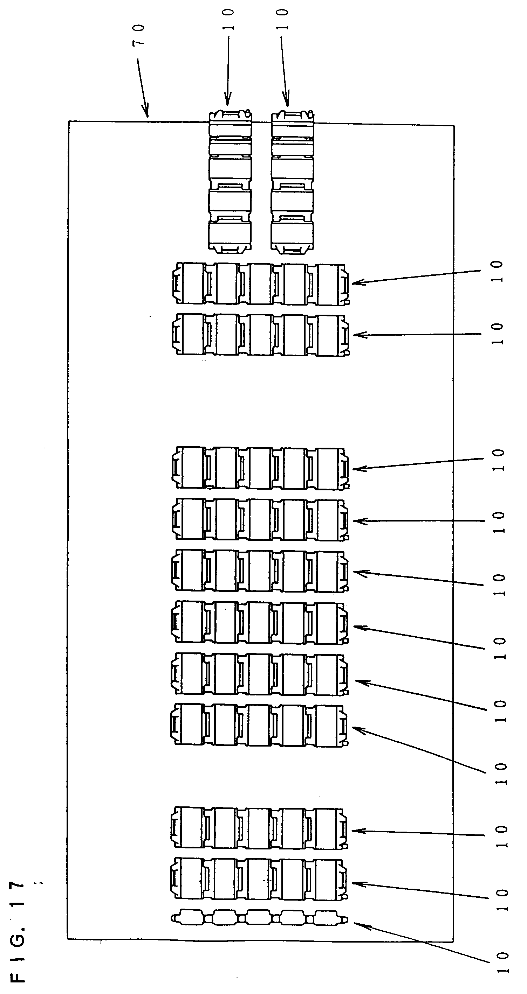

Fig. 16 is a side view showing a state of use of the second embodiment.

Fig. 17 is a plan view showing a magnetic therapeutic apparatus according to the third embodiment of this invention.

Fig. 18 is a side view of the third embodiment.

### Best Mode for Carrying Out the Invention

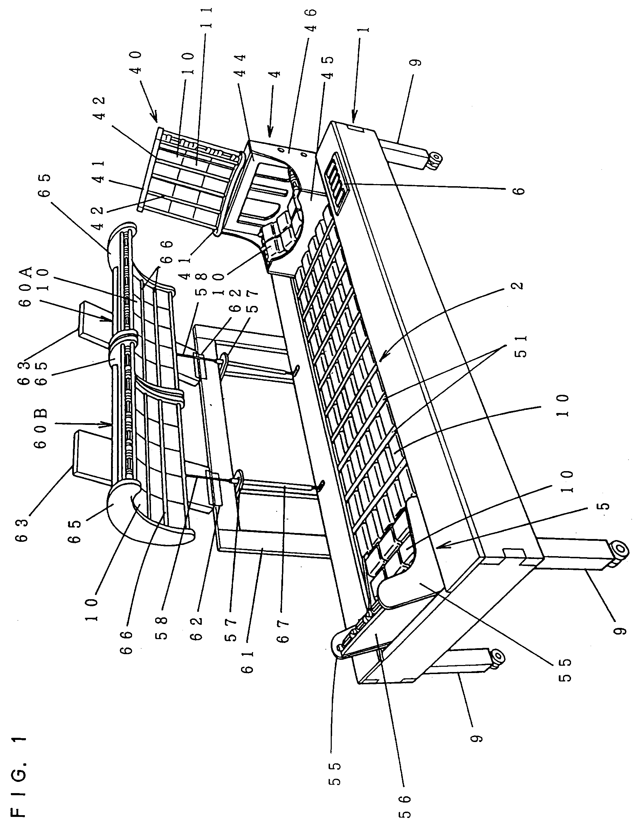

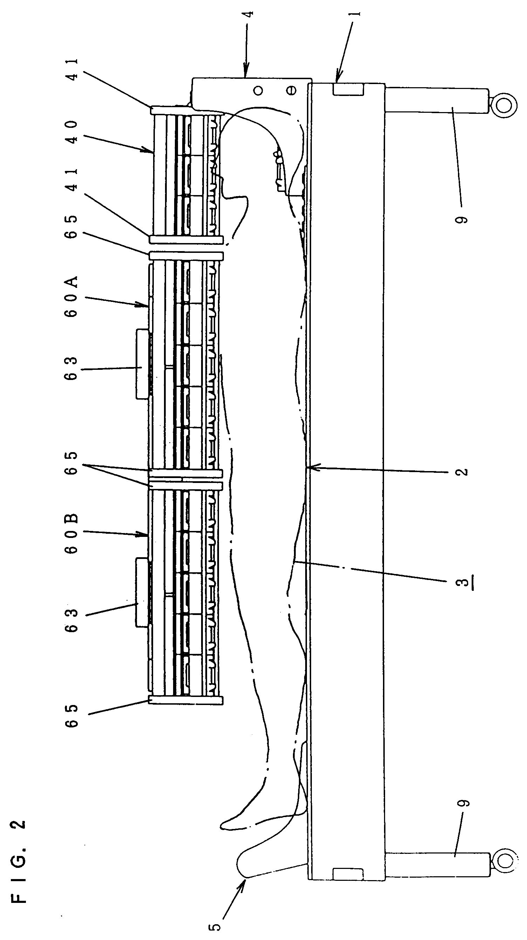

Figs. 1 and 2 show a whole structure of a magnetic therapeutic apparatus according to first embodiment of this invention.

The magnetic therapeutic apparatus of the figures comprises a bed 1 having a horizontal support face 2 with a sufficient area on which a human body 3 can lie and three covers 40, 60A and 60B disposed above the bed 1. Respective portions on said bed 1 and the three covers 40, 60A and 60B are provided with many magnetic field generators 10, and magnetic fields produced by the magnetic field generators 10 are exerted on the whole body 3. In addition, to the four corners of the bed 1, legs 9 having castors at their bottoms are attached.

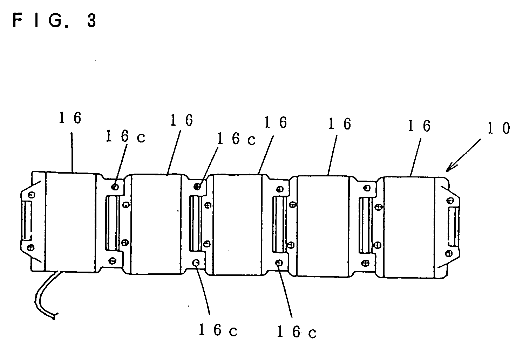

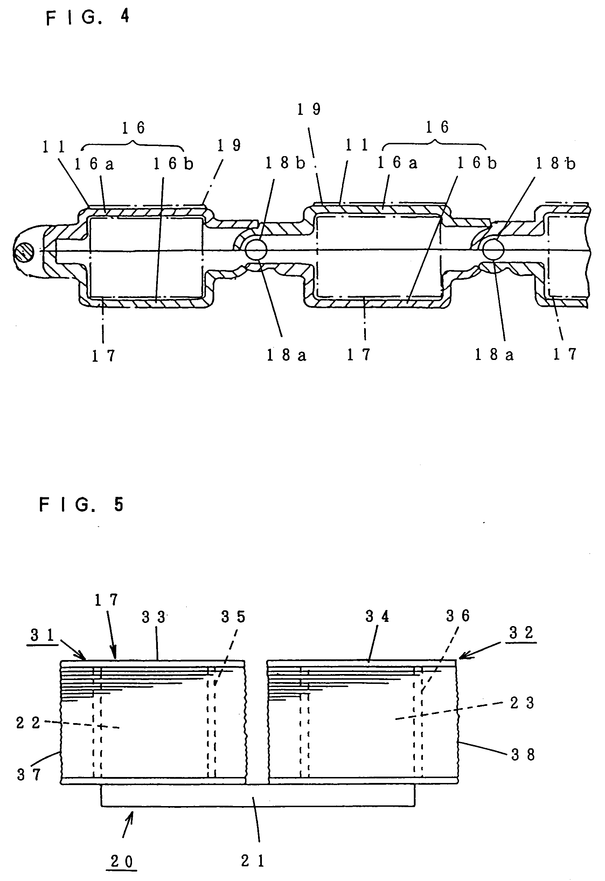

As shown in Figs. 3 and 4, said each magnetic field generator 10 is comprised of a plurality of synthetic resin cases 16 each housing solenoids 17 therein. Each case 16 is formed by butting the openings of the paired halves 16a and 16b of the case and fixing plural portions with screws 16c. On the side end face of each case 16, either protruding axes 18a and 18a extending in the direction of the width of each case 16 or axis receiving holes 18b and 18b are formed, and a case 16 and an adjoining case 16 are pivotally rotatably connected with each other by inserting the protruding axis 18a into the axis receiving hole 18b.

As shown in Fig. 5, said solenoid 17 comprises a laminated iron core 20 provided with legs 22 and 23 at both ends of a base 21 and coil bobbins 31 and 32 engaged with the legs 22 and 23 respectively. Each coil bobbin 31 or 32 is constituted by winding a coil 37 or 38 on periphery of a spool 35 or 36 with a flange 33 or 34 made of synthetic resin, and when A.C. current is flowed through the coils 37 and 38, alternating magnetic fields are generated from the ends of the legs 22 and 23. The solenoid 17 is fixed in the case 16, with the end faces of said legs 22 and 23 being oriented to one half 16a of the case, and this makes the outer face of said one half 16a a magnetic pole face 11 which generates magnetic fields.

In addition, on the magnetic pole face 11 of the one half 16a, a radiation coating film 19 may be formed, by coating the face with a far infrared radiation material such as zirconia, zircon, titania, alumina, cordierite or quatzite, as required. This far infrared radiation material is excited by heat to radiate for infrared rays. Besides coating the case 16 surface with the far infrared radiation materials, these far infrared radiation materials may be mixed in a molding material of the case 16.

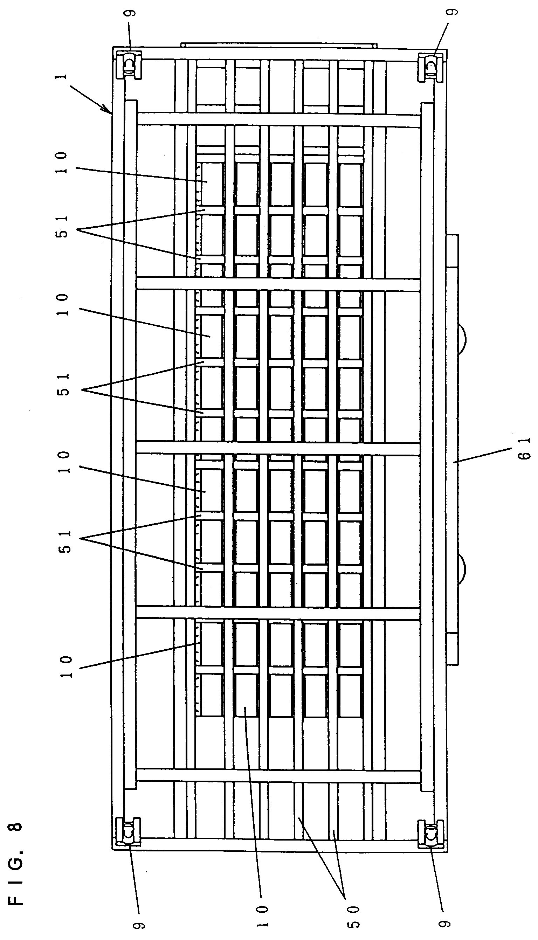

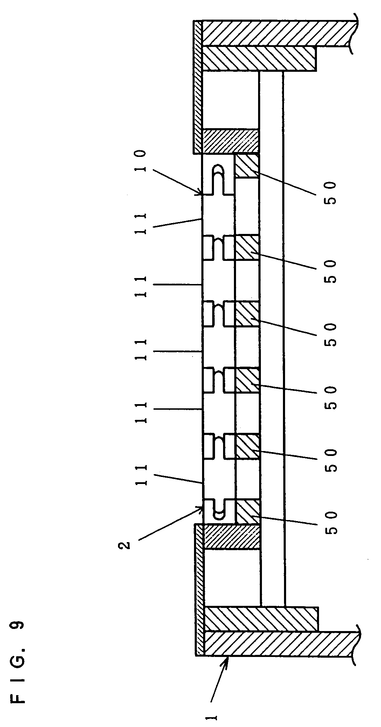

As shown in Fig. 9, plurality of the magnetic field generators 10 are arranged in parallel on the horizontal support face 2 of said bed 1, with the magnetic pole faces 11 facing upward. As shown in Figs. 8 and 9, said horizontal support face 2 is structured by disposing a plurality of longitudinal bars 50 in equal space, and each magnetic field generator 10 is so supported on the horizontal support face 2 that its connecting portions are engaged with respective longitudinal bars 50. On said horizontal support face 2, a plurality of transverse bars 51 are arranged in equal space, in the direction of the width perpendicular to the longitudinal bars 50. Each transverse bar 51 is interposed between adjacent magnetic field generators 10 and 10, by which respective magnetic field generators 10 are positioned and fixed.

One end of said horizontal support face 2 (right end in Fig. 1) is provided with a head support 4 for supporting a head of a human body 3, and above this head support 4, said cover 40 is disposed.

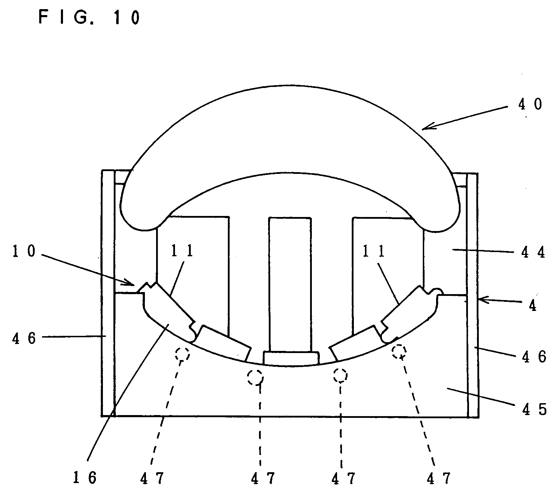

As shown in Fig. 10, said head support 4 comprises opposite front plate 44 and rear plate 45, opposite side plates 46 and 46, and a plurality of bars 47 disposed between said front plate 44 and said rear plate 45. Among these bars 47, bars 47 placed in the center of the width are disposed in lower positions, while bars 47 placed at both ends are disposed in higher positions respectively. On each bar 47, two magnetic field generators 10 are arranged in parallel, with the magnetic pole faces 11 facing upward and in the curved state. Each magnetic field generator 10 is so supported that its connecting portions are engaged with the bar 47.

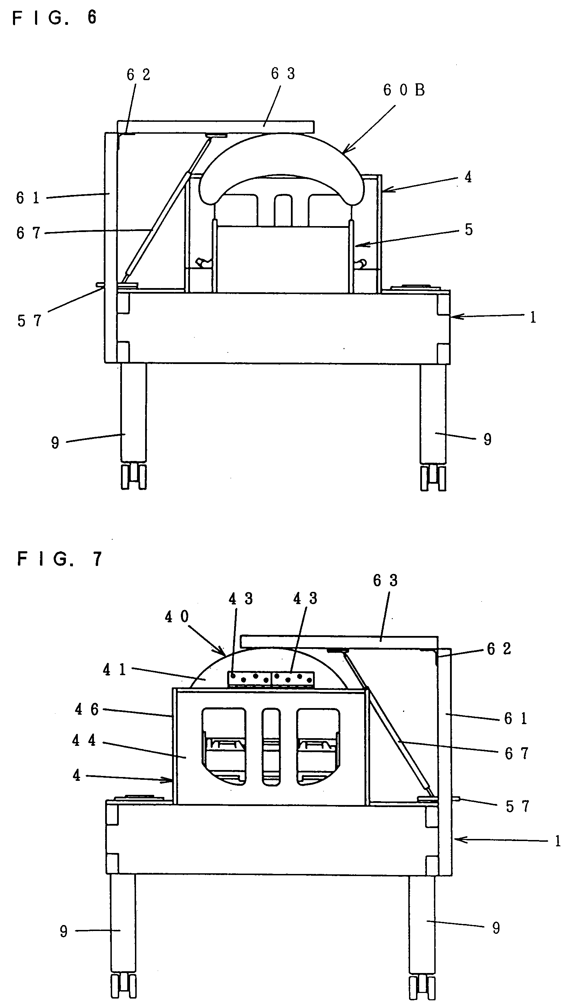

As shown in Figs. 1 and 7, said cover 40 is attached, with being freely lifted up and down and being freely risen and lain, to the upper end of said front plate 44 by hinges 43 and 43. This cover 40, when turned to the side of the bed 1, is held horizontal to be positioned oppositely to and above said head support 4, and the cover 40, when erected, is supported on the front plate 44, in the standing-up state.

This cover 40 comprises front and rear end plates 41, 41 and a plurality of bars 42 disposed between these plates 41. Among these bars 42, bars 42 placed in the center of the width are disposed in higher positions, while bars 42 placed at both ends are disposed in lower positions respectively. On each bar 42, three magnetic field generators 10 are arranged in parallel, with the magnetic pole faces 11 facing downward and in the curved state. Each magnetic field generator 10 is so supported that its connecting portions are engaged with the bar 42.

The other end of said horizontal support face 2 (left end in Fig. 1) is provided with a feet support 5 for supporting the feet of the human body 3.

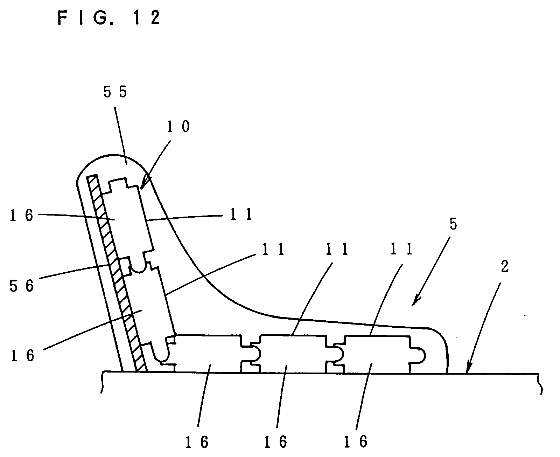

As shown in Fig. 12, this feet support 5 comprises opposing side plates 55 and 55 and a back plate 56 provided between both the side plates 55 and 55, and three magnetic field generators 10 are disposed in parallel in the direction of the width, ranging from the upper face of the bed 1 toward the back plate 56, with the magnetic pole faces 11 facing upward and in the curved state. Each magnetic field generator 10 is disposed in such a manner that two cases 16 are positioned along the back plate 56 and three cases are positioned along the upper face of the bed 1 respectively.

As shown in Figs. 1, 6 and 7, one side edge of the bed 1 is equipped with a gate shape support 61, and wooden arms 63 and 63 are attached, with being freely lifted up and down and being freely risen and lain, to the front and rear positions of the upper edge of this support 61, using hinges 62 and 62. At the end of each arm 63, said cover 60A or 60B is hung by a hanging hook (not shown). When the arms 63 and 63 are turned to the side of the bed 1, each cover 60A or 60B is held horizontal by a rod-like support device 67. When the arms 63 are erected, the support device 67 is held by a support plate 57 and the arm 63 is held on the support 61, in the standing-up state.

Said support device 67 has a rod 58 which can perform telescopic operation, and lower end thereof is connected to the upper face of the bed 1. The end of the rod 58 is linked to the longitudinal center of said arm 63. In addition, said support plate 57 is a ring plate having a hole, and is vertically movable along the support device 67.

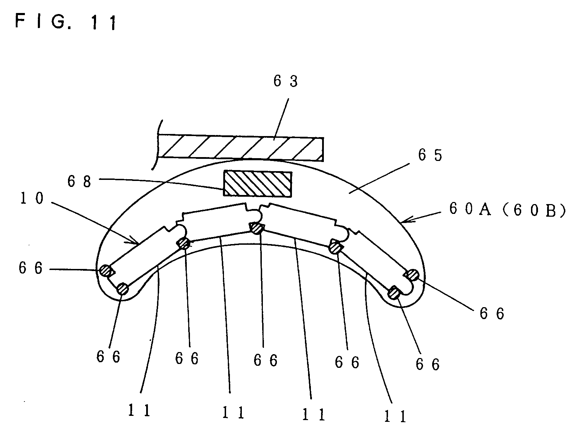

As shown in Fig. 11, each of said cover 60A, 60B is comprised of front and rear end plates 65 and 65, a plurality of bars 66 disposed between these end plates, and a connecting plate 68. Among said bars 66, bars 66 placed in the center of the width are disposed in higher positions, while bars 66 placed at both ends are disposed in lower positions respectively. On each bar 66, five magnetic field generators 10 are arranged in parallel, with the magnetic pole faces 11 facing downward and in the curved state. Each magnetic field generator 10 is so supported that its connecting portions are engaged with the bar 66.

On the bed 1, an operation control portion 6 comprised of a plurality of power supply switches, is provided. The power supply switches of the operation control portion 6 are for respectively supplying or stopping electric power to the magnetic field generators equipped on the horizontal support face 2, in the head support 4, the feet support 5 and the covers 40, 60A and 60B.

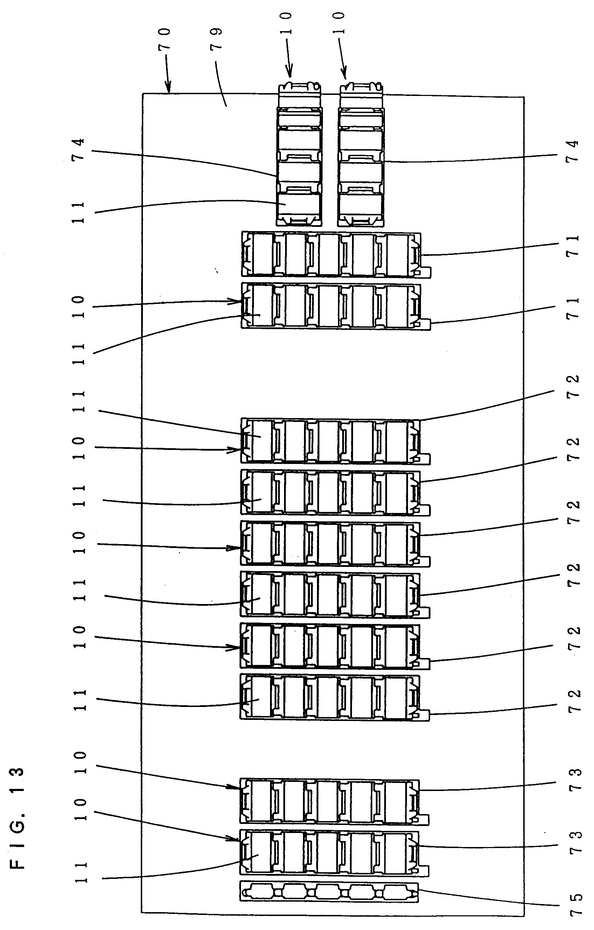

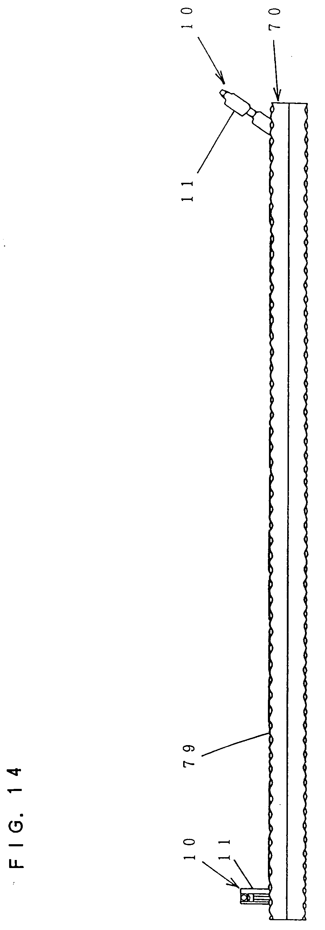

Fig. 13 and 14 show the second embodiment of this invention, in which a plurality of the magnetic field generators 10 are arranged in parallel in a cushion mat 70.

Said cushion mat 70 has a horizontal support face 79 large enough for a human body to lie, and this horizontal support face 79 is provided with two transverse recesses 71 and two longitudinal recesses 74 in the position of supporting a human head, six transverse recesses 72 in the position of supporting a human body trunk, and furthermore three recesses 73 and 75 in the position of holding human feet.

Among these recesses 71 - 75, in the recesses 71 - 73 magnetic field generators 10 are housed, with the magnetic pole faces 11 of them facing upward, and in the recess 75 a magnetic field generator 10 is housed, with their magnetic pole faces 11 facing horizontally. The magnetic field generators 10 housed in said recesses 71 - 73 are aligned on the same face as the horizontal support face 79, while the magnetic field generator 10 housed in said recess 75 projects from the horizontal support face 79.

In said recesses 74, magnetic field generators 10 are arranged with the magnetic pole faces 11 of them facing upward and in the curved state, and two cases 16 of said magnetic field generators 10 project obliquely from the horizontal support face 79.

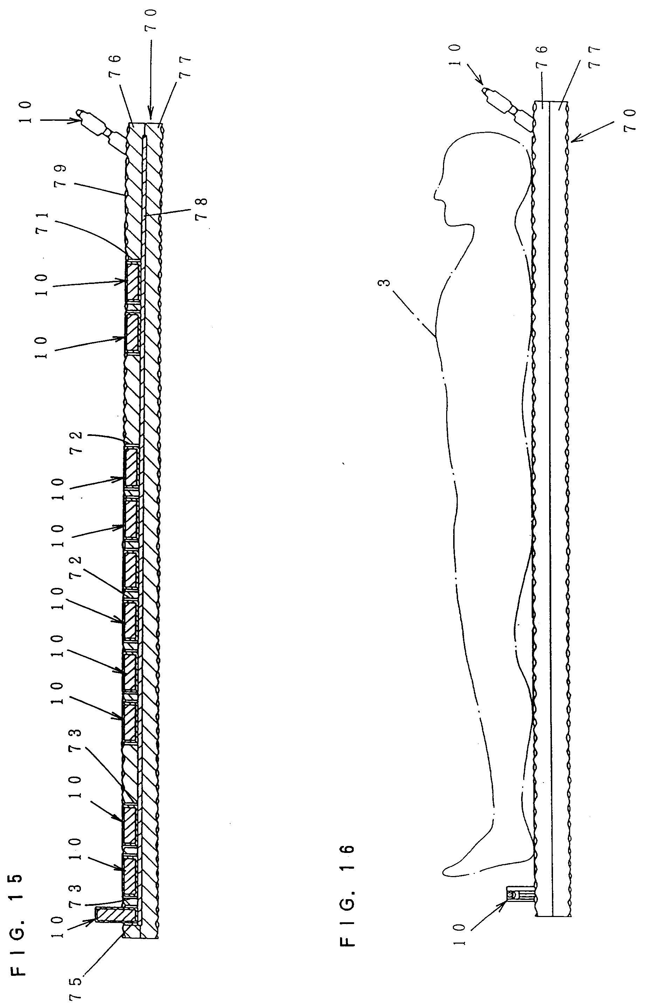

As shown in Fig. 15, said cushion mat 70 is comprised of a first semi-rigid cushion material 76 provided with corrugated faces over its whole surface, a second cushion material 77 of the same material qualities, and a corrugated plate 78 of synthetic resin, integrally joining both the cushion materials 76, 77 as the corrugated plate 78 intervening between them. Said respective recesses 71 - 75 are formed in the first cushion material 76, and the magnetic field generators 10 housed in the respective recesses 71 - 75 are held on said corrugated plate 78.

In addition, the whole mat 70 is wrapped by a cloth cover.

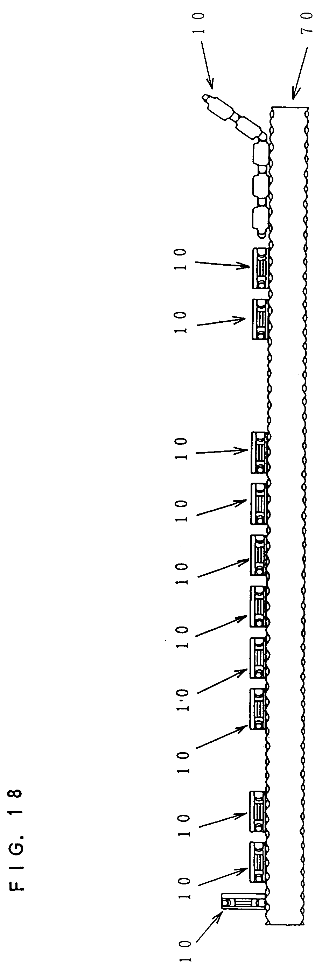

Figs. 17 and 18 show a magnetic therapeutic apparatus according to the third embodiment of this invention. In this embodiment, each magnetic field generator 10 is disposed on the upper face of the cushion mat 70 and fixed by a face fastener or the like. In addition, the arrangement position and the poses of the magnetic field generators 10 and the structure of the cushion mat 70 are the same as the above second embodiment.

### Industrial Applicability

When using the magnetic therapeutic apparatus of the first embodiment, first, the covers 40, 60A and 60B are raised to open, then a patient lies on the horizontal support face 2 of the bed 1, placing his head on the head support 4, and resting his feet on the feet support 5. Then, as shown in Fig. 2, the covers 60A and 60B are put on the chest and abdomen of the body 3, and the cover 40 is put on the head of the body 3. Whereby, the whole body 3 is surrounded by the magnetic field generators 10. Then the respective power supply switches of the operation control portion 6 are turned on, and current flows through the respective coils 37 and 38 of each magnetic field generator 10, and superposed magnetic flux of the coils 37 and 38 is generated from the end faces of the legs 22 and 23 of the laminated iron core 20, and the magnetic fields from the magnetic pole faces 11 are exerted on respective portions of the body 3. In addition, vibration and heat are generated from the iron core 20 and the coils 37 and 38, and the vibration and heat are transmitted to the case 16. As a result, this provides the body 3 with a massaging effect as well as a warming effect.

In the second and the third embodiment, there is no covers of the 40, 60A and 60B, thus any magnetic field does not act from upside.

## Claims

1. A magnetic therapeutic apparatus comprising a mounting base and a plurality of magnetic field generators disposed in parallel on the said mounting base, with their magnetic pole faces aligning,
wherein said each magnetic field generator is so structured that a plurality of cases each housing solenoids therein are connected flexibly with each other in one row, with the magnetic pole faces orienting in the same direction.

2. A magnetic therapeutic apparatus according to claim 1, wherein said mounting base is comprised of a bed having an area sufficient for a human body to lie thereon and covers disposed above the bed, and on said bed a plurality of magnetic field generators are disposed in parallel with their magnetic pole faces orienting upward, while on said each cover a plurality of magnetic field generators are disposed in parallel, with their magnetic pole faces orienting downward.

3. A magnetic therapeutic apparatus according to claim 1 or 2, wherein said covers are attached to support members and front plates vertically fixed to said bed, in such a manner that the covers can be lifted up and down, and raised and lain.

4. A magnetic therapeutic apparatus according to claim 1, wherein said mounting base is a cushion mat having an area sufficient for a human body to lie thereon, and a plurality of recesses are formed on an upper face of the cushion mat, and magnetic field generators are housed in the respective recesses.

5. A magnetic therapeutic apparatus according to claim 1, wherein said mounting base is a cushion mat having an area sufficient for a human body to lie thereon, and a plurality of magnetic field generators are disposed on the upper face of the cushion mat.
